# EUROPEAN PATENT APPLICATION

(11) **EP 4 566 603 A1**
(43) Date of publication of application: **11.06.2025**
(21) Application number: 23214309.9
(22) Date of filing: 05.12.2023
(51) Int. Cl.: A61K 31/4045, A61P 25/20

(54) **USE OF MELATONIN AGONISTS IN CARDIAC DISEASE**

(71) Applicant: Technische Universität München, 80333 München (DE)
(72) Inventor: The designation of the inventor has not yet been filed
(74) Representative: Weickmann & Weickmann PartmbB

(57) **Abstract**

The present invention relates the use of a melatonin agonist in order to prevent and/or ameliorate sleep disorders in a subject suffering from a cardiac disease associated with damage in a peripheral ganglion.

## Description

The present invention relates to the use of a melatonin agonist in order to prevent and/or ameliorate sleep disorders in a subject suffering from a cardiac disease associated with damage in a peripheral ganglion.

### BACKGROUND

About 40-70 % of human cardiac insufficiency (heart failure) patients suffer from disturbances of the sleep-wake cycle (Jaarsmaa et al, 1999; Zheng et al, 2021; Redeker et al, 2008; Parati et al. 2016). Effective therapies, e.g., administration of benzodiazepines, are associated with a high risk of side effects such as drug addiction. Thus, there is a high medical need for therapies with a low risk of side-effects.

Melatonin synthesis occurs in the pineal gland and is, together with its secretion, tightly controlled by sympathetic neurons, which project from the superior cervical ganglia (SCG). The present inventors found that melatonin secretion from the pineal gland is disturbed in a sub-group of patients suffering from cardiac disease due to a possibly irreversible immune-mediated destruction of a specific subset of sympathetic neurons in the SCG that control diurnal rhythmicity of pineal melatonin secretion. These data elucidate defective sympathetic control of the pineal gland to underlie the disturbance of circadian rhythmicity in cardiovascular disease. These results are already described in co-owned application PCT/EP2023/065279, the content of which is herein incorporated by reference.

### SUMMARY OF THE INVENTION

A first aspect of the present invention relates to a melatonin agonist for use in preventing and/or ameliorating a sleep disorder in a patient suffering from a cardiac disease associated with a dysfunction of the superior cervical ganglion (SCG).

A further aspect of the present invention relates to a method of preventing and/or ameliorating a sleep disorder in a patient suffering from a cardiac disease associated with a dysfunction of the superior cervical ganglion (SCG), wherein a therapeutically effective amount of a melatonin agonist is administered to said patient.

The inventors found that a disturbed melatonin secretion is causal for sleep disorders in certain cardiac patients and identified a novel sub-group of patients which can be treated effectively by administering a melatonin agonist.

The novel sub-group of patients is characterized by an SCG dysfunction, particularly by an increased size of the SCG.

### DETAILED DESCRIPTION

According to the present invention, sleep disorders, e.g., sleep disturbances occurring in a cardiac patient, particularly a human cardiac patient, can be treated effectively by administering a melatonin agonist. Thereby, a substantive improvement in quality of life can be achieved.

In certain embodiments, the patient eligible for the treatment is suffering from a cardiac disease, wherein the cardiac disease may be selected from hypertension, e.g., grade II and/or grade III, myocardial infarction, e.g., post-acute treatment phase, heart failure, heart valve disease, e.g., moderate grade and/or severe grade, cardiomyopathy including but not limited to dilatative and hypertrophic cardiomyopathy, cardiac arrhythmia, coronary artery disease, and any combination thereof. In particular embodiments, the cardiac disease is congestive heart failure of NYHA class I, II, III or IV, particularly congestive heart failure of NYHA class II or III.

The patient eligible for treatment suffers from a sleep disorder, e.g., a disturbance of the sleep-wake cycle, or is at risk from acquiring a sleep disorder. In certain embodiments, the sleep disorder may be characterized by difficulty in initiating and maintaining sleep, insomnia, sleep fragmentation, nonrestorative sleep, prolonged sleep latency, poor sleep quality and continuity, excessive daytime fatigue, periodic limb movements during sleep or any combination thereof. In certain embodiments, the patient does not suffer from obstructive sleep apnea. In other embodiments, the patient suffers from obstructive sleep apnea.

According to the present invention, the patient eligible for treatment is characterized by the presence of a novel biomarker identified by the present inventors. The novel biomarker is an SCG dysfunction.

In certain embodiments, the dysfunction is an SCG hypertrophy which may be characterized by an increase in ganglion size (measured as length and/or volume) of more than about 50% or of more than about 25%. In certain embodiments, the dysfunction is an increased length of the SCG which may be determined by using ultrasound imaging optionally in combination with echocardiography (ECG). In particular embodiments, the patient is characterized by an increased SCG length of at least 2.5 cm, particularly of at least 3.0 cm. In contrast thereto, the average SCG length of a healthy control person is 2.175 cm (SD of ± 0.2712; 95% CI interval from 1.948 to 2.402 cm) (Ziegler et al.; 2023).

In certain embodiments, the SCG dysfunction is a disrupted ganglion activity which may be indicated by increased periodic repolarization dynamics, e.g., assessed by 3D high-resolution resting ECGs and/or ganglion inflammation, e.g., indicated by an increased number of immune cells such as macrophages.

In certain embodiments, the patient is further characterized by a disturbed melatonin secretion, which is diminished by 50% or more, particularly by 75% or more as compared to typical time-corrected levels in heart-healthy specimens (Brugger et al., 1995). The melatonin secretion may be determined in bodily fluid samples, e.g., plasma and/or urine samples from the patient.

According to the present invention, the patient is treated with a melatonin agonist. The term "melatonin agonist" relates to melatonin, melatonin analogs or further compounds which show a melatonin-agonistic activity, e.g., by binding to the melatonin receptor and mediating at least partially the physiological effects of melatonin. In certain embodiments, the melatonin agonist is selected from a group consisting of melatonin and a melatonin analog including but not limited to Agomelatin, Ramelteon, and Tasimelteon.

The melatonin agonist may be administered in a therapeutically effective amount by any suitable administration route, e.g., orally, nasally etc. The amount depends on the specific melatonin agonist used, the administration route and the severity of the sleeping disorder. Typically, the melatonin agonist is administered at least once daily. The melatonin agonist may be in a fast-acting dosage form, a retard dosage form or a combination thereof.

In certain embodiments, administration of a melatonin agonist may be as follows:
Ramelteon (Rozerem^{®}) is administered at a recommended dose of about 4-24 mg daily, e.g., 8 mg daily. The medicament may be taken within 30 minutes of going to bed.
Tasimelteon (Hetlioz^{®}) is administered at a recommended dose of about 4-50 mg daily, e.g., 20 mg daily. The medicament may be taken about 1 hour before bedtime, at the same time every night.
Agomelatin (Valdoxan^{®}) is administered at a recommended dose of about 5 to 75 mg daily, e.g., 25 mg daily with a possible increase to 50 mg daily.
Melatonin (Circadin^{®}) is administered at a recommended dose of about 1 to 10 mg daily, e.g., 2 mg daily with a possible increase to 5 mg daily.

In certain embodiments, administration of a melatonin agonist allows the patient to avoid administration of other sleeping drugs, particularly to avoid administration of addictive medicaments such as benzodiazepines or barbiturates.

In certain embodiments, the patient is being administered at least one further medicament in addition to the melatonin agonist.

For example, the at least one further medicament may be a standard medicament for the treatment of cardiac disease such as a beta blocker. Typically, such a standard medicament is continuously administered to the patient throughout the treatment with the melatonin agonist.

Alternatively or additionally, an anti-inflammatory agent may be administered for preventing damage, e.g., degeneration and/or death of cells in the SCG. As described in PCT/EP2023/065279, the anti-inflammatory agent may be any pharmaceutically acceptable anti-inflammatory agent, particularly any pharmaceutically acceptable anti-inflammatory agent capable of counteracting inflammatory processes mediated by, and/or associated with pro-inflammatory cells, e.g., pro-inflammatory macrophages.

For example, the anti-inflammatory agent may be a steroidal drug, an immunoglobulin preparation, an inhibitor of the complement cascade, an immunomodulator, and/or an antagonist of a chemokine, cytokine, or colony-stimulating factor. In certain embodiments, the anti-inflammatory agent is a biological, e.g., a monoclonal antibody, a recombinant fusion protein or a nucleic acid therapeutic.

In certain embodiments, the anti-inflammatory agent may be selected from:
- a glucocorticoid, including but not limited to Dexamethasone;
- an immunoglobulin preparation, including but not limited to IVIg;
- a complement inhibitor, including but not limited to a C3 antagonist such as Compstatin or Pegcetacoplan, or a C5 antagonist such as Eculizumab, Zilucoplan, Tesidolumab, or SKY59;
- a nucleic acid therapeutic including but not limited to an miR-21 inhibitor;
- an immunomodulator, including but not limited to Alemtuzumab, Mitoxantron, or Orcrelizumab; and
- an antagonist of a chemokine, cytokine or colony-stimulating factor including but not limited to Etanercept, Adalimumab or PLX5622; and
- a macrophage inhibitor including but not limited to a bisphosphonate compound such as clodronate.

In particular embodiments, the anti-inflammatory agent is an inhibitor of miR-21 which is coupled to a moiety that delivers the inhibitor of miR-21 to a macrophage as described in WO 2021/205032, the content of which is herein incorporated by reference. In more particular embodiments, the inhibitor of miR-21 is a modified nucleic acid molecule, e.g., a phosphorothioate-modified locked nucleic acid (LNA) oligonucleotide molecule, which hybridizes with miR-21 and/or the moiety that delivers the inhibitor of miR-21 to a macrophage is a trimannose moiety. In even more particular embodiments, the inhibitor of miR-21 is the trimannose-coupled, phosphorothioate-modified LNA oligonucleotide molecule RCS-21 as disclosed in Fig. 2g of C. Beck et al. (2023), the content of which is herein incorporated by reference.

In certain embodiments, the anti-inflammatory agent is administered locally to the SCG, e.g., by intraganglionic injection.

A further aspect of the present invention relates to the use of an anti-inflammatory agent as herein described above, particularly of an inhibitor of miR-21, for the treatment of chronic obstructive sleep apnea, particularly chronic obstructive sleep apnea associated with or caused by a damage of the SCG. According to certain embodiments of this aspect, a melatonin agonist is not administered.

In the following, the present invention shall be explained in more detail be the following Examples.

### EXAMPLES

### 1. Determination of SCG size in human cardiac patients by ultrasound imaging

### 1.1. Methods

Ultrasound imaging of the human superior cervical ganglia was executed after assessment of cardiac function by echocardiography. The superior cervical ganglia were located as previously described (Siegenthaler et al. 2013). In brief, the carotid arteries were followed cranially. The superior cervical ganglia are located at level of vertebral body C3, anterior to the longus capitis muscle and dorsal to the internal carotid artery. After visualization of the left ganglion, total ganglionic length was assessed. For ultrasound imaging, a Philips EPIQ 7G device was used. The X5-1 transducer was used for echocardiography and the L12-3 linear transducer for SCG ultrasound imaging. This clinical study was in accordance with the Declaration of Helsinki and approved by the local Ethics Committee.

### 1.2. Results

In a clinical study, the inventors evaluated SCG dimensions by quantitative ultrasound imaging in heart failure patients and healthy controls. Evaluation with current clinical ultrasound equipment revealed a significant increase of SCG dimensions in patients with heart failure compared to heart healthy controls with eight out of nine patients displaying an approx. 150% longer SCG length. In line with a functional interdependence, a significant correlation between SCG size and ejection fraction was observed.

### 2. Clinical Study Protocol

A double-blind study is performed with human cardiac patients to test the efficiency of administering melatonin or a melatonin agonist (active drug) for treating a sleep disorder in a human cardiac patient. The study participants receive randomly the active drug or a placebo.

The active drug is:
(i) the melatonin agonist Ramelteon (e.g., the product Rozerem^{®}) to be administered at a recommended dose of 8 mg daily and to be taken within 30 minutes of going to bed, or
(ii) melatonin (e.g., the product Circadin^{®}) to be administered at a recommended dose of 5 mg daily.

Patient inclusion criteria:
- Age: 35-75 years;
- SCG size increase (length of at least 2.5 cm and particularly of at least 3.0 cm as determined by ultrasound imaging and optionally echocardiography);
- Diagnosis: congestive heart failure of NYHA class II or III;
- Concurrent treatment with a beta blocker.

Patient exclusion criteria:
- Diagnosis: obstructive sleeping apnea.

The study is conducted for 5 to 6 weeks. The patients mainly stay at home and fill out daily sleep diaries. Regular measurements of blood pressure and physical activities are carried out. During that time, the patients visit the hospital three times where plasma and urine samples are taken to determine the concentration of melatonin/melatonin agonist. Further, the patients fill out questionnaires.

### REFERENCES

T. Jaarsma , R. Halfens, H. H. Abu-Saad, K. Dracup, J. Stappers and J. van Ree. Quality of life in older patients with systolic and diastolic heart failure. Eur J Heart Fail. 1999; 1(2):151-60. doi: 10.1016/s1388-9842(99)00007-0.
T. Zheng. Sleep disturbance in heart failure: A concept analysis. Nurs Forum. 56(3):710-716 (2021). doi: 10.1111/nuf.12566.
N. S. Redeker. Sleep disturbance in people with heart failure: implications for self-care. J Cardiovasc Nurs. 23(3):231-8 (2008). doi:10.1097/01. JCN.0000305094.20012.76.
G. Parati, C. Lombardi, F. Castagna, P. Mattaliano, P. Perrone Filardi and P. Agostoni. Heart failure and sleep disorders. Nat Rev Cardiol. 13(7):389-403 (2016). doi: 10.1038/nrcardio.2016.71.
A. Siegenthaler, M. Haug, U. Eichenberger, M. R. Suter, B. Moriggl, Block of the Superior Cervical Ganglion, Description of a Novel Ultrasound-Guided Technique in Human Cadavers. Pain Med. (United States) 14, 646-649 (2013).
P. Brugger, W. Marktl, M. Herold. Impaired nocturnal secretion of melatonin in coronary heart disease. The Lancet. 345(8962):1408 (1995). doi: 10.1016/S0140-6736(95)92600-3.
C. Beck et al. Trimannose-coupled antimiR-21 for macrophage-targeted inhalation treatment of acute inflammatory lung damage. Nature Communications 14: 4564 (2023).
K. Ziegler et al. Immune-mediated denervation of the pineal gland underlies sleep disturbance in cardiac disease. Science 381: 285-290 (2023). doi: 10.1126/science.abn6366.

## Claims

1. A melatonin agonist for use in preventing and/or ameliorating a sleep disorder in a patient suffering from a cardiac disease associated with a dysfunction of the superior cervical ganglion (SCG).

2. A melatonin agonist for the use of claim 1, which is selected from the group consisting of melatonin and melatonin analogs comprising Agomelatin, Ramelteon, and Tasimelteon.

3. The melatonin agonist of claim 1 or 2 for the use of claim 1 or 2, wherein the patient is a human patient.

4. The melatonin agonist of claim 1 or 2 for the use of any one of claims 1 to 3, wherein the sleep disorder is a disturbance of the sleep-wake cycle.

5. The melatonin agonist of claim 1 or 2 for the use of any one of claims 1 to 4, wherein the cardiac disease is hypertension, myocardial infarction, heart failure, heart valve disease, cardiomyopathy, cardiac arrhythmia, coronary artery disease, or any combination thereof.

6. The melatonin agonist of claim 1 or 2 for the use of any one of claims 1 to 5, wherein the cardiac disease is congestive heart failure of NYHA class I, II, III or IV, particularly congestive heart failure of NYHA class II or III.

7. The melatonin agonist of claim 1 or 2 for the use of any one of claims 1 to 6, wherein the patient is further **characterized by** a disturbed melatonin secretion.

8. The melatonin agonist of claim 1 or 2 for the use of any one of claims 1 to 7, wherein the SCG dysfunction is an increased ganglion size.

9. The melatonin agonist of claim 1 or 2 for the use of any one of claims 1 to 8, wherein the SCG dysfunction is an increased ganglion length of at least 2.5 cm, particularly of at least 3.0 cm.

10. The melatonin agonist of claim 1 or 2 for the use of claim 8 or 9, wherein the SCG dysfunction is measured using ultrasound imaging optionally in combination with echocardiography.

11. The melatonin agonist of claim 1 or 2 for the use of any one of claims 1 to 10, wherein at least one further medicament is administered to the patient.

12. The melatonin agonist of claim 1 or 2 for the use of claim 11, wherein the at least one further medicament is a medicament for treating the cardiac disease, particularly a beta blocker.

13. The melatonin agonist of claim 1 or 2 for the use of claim 11 or 12, wherein the at least one further medicament is an anti-inflammatory agent for preventing damage, e.g., degeneration and/or death of cells in the SCG.

14. A method of preventing and/or ameliorating a sleep disorder in a patient suffering from a cardiac disease associated with a dysfunction of the superior cervical ganglion (SCG), wherein a therapeutically effective amount of a melatonin agonist is administered to said patient.
